**Europäisches Patentamt**

**European Patent Office**

**Office européen des brevets**

(19)

(11) Veröffentlichungsnummer: **0 249 681**
**B1**

## (12) EUROPÄISCHE PATENTSCHRIFT

(45) Veröffentlichungstag der Patentschrift:
**27.12.90**

(21) Anmeldenummer: **87102951.8**

(22) Anmeldetag: **02.03.87**

(51) Int. Cl.⁵: **A61N 1/362**

(54) Messvorrichtung zur intrakardialen Erfassung der Blutsauerstoffsättigung.

(30) Priorität: **16.06.86 DE 3620279**

(43) Veröffentlichungstag der Anmeldung:
**23.12.87 Patentblatt 87/52**

(45) Bekanntmachung des Hinweises auf die Patenterteilung:
**27.12.90 Patentblatt 90/52**

(84) Benannte Vertragsstaaten:
**DE FR GB IT NL SE**

(56) Entgegenhaltungen:
**EP-A- 0 059 868**

(73) Patentinhaber: **Siemens Aktiengesellschaft,
Wittelsbacherplatz 2, D-8000 München 2(DE)**

(84) Benannte Vertragsstaaten: **DE FR GB IT NL**

(73) Patentinhaber: **Siemens Elema AB, Röntgenvägen 2,
S-171 95 Solna 1(SE)**

(84) Benannte Vertragsstaaten: **SE**

(72) Erfinder: **Heinze, Roland, Dipl.-Ing., Simbacherstrasse 9,
D-8000 München 80(DE)**
Erfinder: **Elmqvist, Hakan, Dr., Sunnerdahlsvägen 7,
S-16138 Bromma(SE)**

## Beschreibung

Die Erfindung betrifft eine Meßvorrichtung zur intrakardialen Erfassung der Blutsauerstoffsättigung zur Frequenzregelung eines Herzschrittmachers mit einer Meßsonde, die einen Lichtsender und einen Lichtempfänger enthält, wobei der Lichtempfänger das vom Lichtsender ausgesandte und vom Blut reflektierte Licht empfängt und wobei Lichtsender und Lichtempfänger parallel über zwei gemeinsame Leitungen an eine Auswerteschaltung angeschlossen sind.

Eine derartige Meßvorrichtung ist aus der DE-PS 3 152 963 bekannt. Dabei enthält die Meßsonde eine Kombination aus einer Leuchtdiode und einem Fototransistor, die derart parallel geschaltet sind, daß der Durchlaßstrom durch die Leuchtdiode additiv überlagert wird von dem durch Lichteinwirkung verursachten Strom durch den Fototransistor. Bei Ansteuerung der Meßsonde mit einem konstanten Strom oder einer konstanten Spannung löst das vom Blut, abhängig von seiner Sauerstoffsättigung, reflektierte Licht im Fototransistor einen Stromfluß aus, der eine Stromänderung bzw. Spannungsänderung an der Meßsonde bewirkt. Die durch Lichtreflexion erzeugte Spannungs- bzw. Stromänderung wird in einer Auswerteschaltung durch Vergleich des Meßsignals bei angesteuerter Leuchtdiode und Fototransistor mit einem Referenzsignal ermittelt.

Dieses Referenzsignal wird dadurch gebildet, daß man über eine der Leuchtdiode antiparallel geschaltete Diode einen Impuls gleicher Spannung, aber umgekehrten Vorzeichens wie für die Nutzsignalmessung schickt. Dabei sind vorzugsweise die Kennlinie der Diode im Referenzkreis und die Kennlinie der Leuchtdiode identisch.

Bei dieser Meßvorrichtung kommt man also sowohl für die Nutzsignalmessung als auch für die Referenzmessung mit nur zwei elektrischen Zuleitungen aus. Dies ist deshalb von besonderer Bedeutung, weil diese Zuleitungen in Kathetern mit möglichst geringem Durchmesser und hoher Flexibilität untergebracht werden müssen und außerdem jede zusätzliche Leitung die Ausfallwahrscheinlichkeit erhöht.

Nachteilig bei der genannten Anordnung ist jedoch, daß zur Referenzmessung eine Umpolung des Meßsignals erforderlich ist. Damit ist beim üblichen Aufbau der Spannungsversorgung von Herzschrittmachern, bei denen ein Pol der Versorgungsspannung fest mit dem Gehäuse verbunden ist, ein nicht unbeträchtlicher Schaltungsaufwand verbunden. Ferner wird für die Referenzmessung derselbe Strom verbraucht wie für die Nutzsignalmessung.

Aufgabe der Erfindung ist es, eine Meßvorrichtung der eingangs genannten Art so auszugestalten, daß eine Referenzmessung unnötig wird.

Diese Aufgabe wird erfindungsgemäß dadurch gelöst, daß in Reihe zum Lichtsender eine Konstantstromquelle angeordnet ist. Damit setzt sich der in die Meßsonde fließende Strom $i_S$ zusammen aus dem Empfangsstrom $i_E$, der die gewünschte Meßgröße enthält und einem - bekannten - Konstantstrom $i_K$. Damit erübrigt sich eine gesonderte Referenzmessung zur Eliminierung der Einflüsse von Temperatur und Zuleitungswiderstand.

Die Konstantstromquelle kann vorteilhafterweise auf unterschiedliche Stromwerte umschaltbar sein. Damit kann die Leistung des Lichtsenders an die Erfordernisse angepaßt werden, beispielsweise bei durch Ablagerungen verstärkter Dämpfung erhöht werden.

In einer vorteilhaften Ausführungsform kann der Lichtsender aus einer Rotlichtdiode und einer Infrarotlichtdiode bestehen, wobei die Wellenlänge des von der Infrarotlichtdiode ausgestrahlten Lichts in einem Bereich liegt, bei dem dessen Reflexion am Blut von der Blut-Sauerstoffsättigung unabhängig ist und wobei die Infrarotlichtdiode und die Rotlichtdiode über einen Umschalter in Reihe mit einer Konstantstromquelle dem Lichtempfänger parallel geschaltet sind. Mit der Infrarotlichtdiode wird eine Referenzmessung durchgeführt, die jedoch nicht wie beim eingangs genannten Stand der Technik zur Eliminierung der Einflüsse von Zuleitungswiderständen und Temperatur dient, sondern zur Kompensation von Ablagerungen an der Meßsonde.

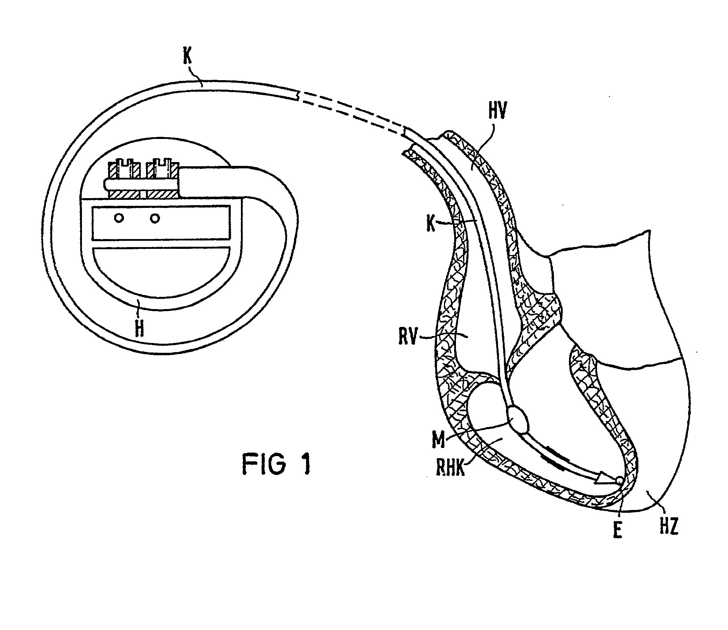

Ausführungsbeispiele der Erfindung werden nachfolgend anhand der Figuren 1 bis 4 näher erläutert.

Figur 1 zeigt die Anordnung eines Herzschrittmachers H, dessen zwei elektrische Leitungen enthaltender Katheter in die obere Hohlvene HV durch den rechten Vorhof RV in die rechte Herzkammer RHK des Herzens HZ geführt wird, wo die Meßsonde M zur Messung der Blutsauerstoffsättigung plaziert wird und durch eine Stimulationselektrode E der Herzmuskel angeregt wird.

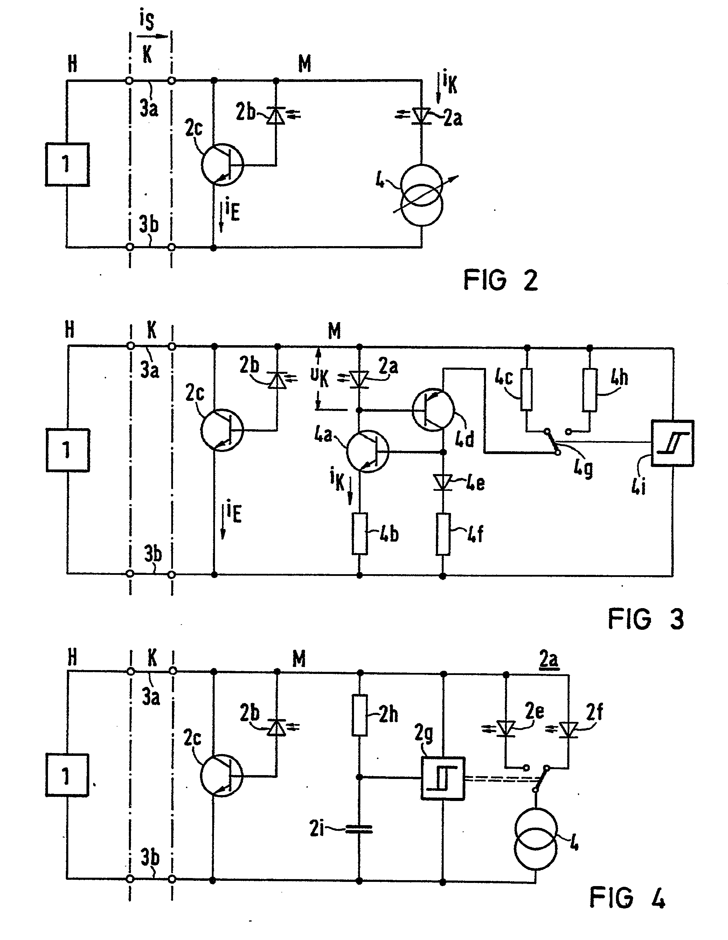

Figur 2 zeigt ein erstes Ausführungsbeispiel für die Meßsonde M. In der Meßsonde M ist die Reihenschaltung eines Lichtsenders in Form einer Rotlichtdiode 2a und einer Konstantstromquelle 4 angeordnet. Ferner enthält die Meßsonde M einen Lichtempfänger, der aus einem Fototransistor 2c mit lichtempfindlicher Kollektor-Basisdiode 2b besteht. Die Kollektor-Emitter-Strecke des Transistors 2c ist der eben beschriebenen Reihenschaltung von Leuchtdiode 2a und Konstantstromquelle 4 parallel geschaltet.

Die Leitfähigkeit des Transistors 2c ist von der Leitfähigkeit der lichtempfindlichen Diode 2b abhängig. Die lichtempfindliche Diode 2b ist so angeordnet, daß sie das vom Lichtsender 2a ausgesandte und vom Blut in Abhängigkeit von dessen Sauerstoffsättigung reflektierte Licht empfängt. Der durch den Lichtempfänger fließende Strom $i_E$ stellt somit ein Maß für die Sauerstoffsättigung des Blutes dar. Der in die Meßsonde M fließende Strom $i_S$ setzt sich zusammen aus dem Empfängerstrom $i_E$ und dem durch den Sender 2a fließenden Strom, der durch die Konstantstromquelle 4 auf einem konstanten Wert $i_K$ gehalten wird. Der als Meßgröße relevante Strom $i_E$ kann daher in der Auswerteschaltung 1 des Herzschrittmachers H auf einfache Weise dadurch ermittelt werden, daß man vom

Gesamtstrom $i_S$ in die Meßsonde M den festen Konstantstrom $i_K$ abzieht. Im Gegensatz zum eingangs erläterten Stand der Technik tritt hier also keine undefinierte Aufteilung des Sondenstroms $i_S$ in den Sende- und Empfangskreis auf, sondern der Strom durch den Empfangskreis ist fest vorgegeben. Damit ist keine gesonderte Referenzmessung zur Eliminierung des Einflusses des Widerstandes der Verbindungsleitungen 3a, 3b des Katheters K und der Temperatur der Meßsonde M notwendig.

Der Konstantstrom der Konstantstromquelle 4 kann einstellbar gemacht werden, was in Figur 2 mit einem Pfeil angedeutet ist. Dies hat den Vorteil, daß man beispielsweise den Sendestrom und damit die Leuchtstärke erhöhen kann, wenn im Laufe der Zeit aufgrund von Ablagerungen an der Meßsonde eine größere Dämpfung auf dem Übertragungsweg auftritt. Die Änderung des Konstantstroms $i_K$ wird dabei zweckmäßigerweise von der Auswerteschaltung 1 vorgegeben und bei der Ermittlung des Meßstroms berücksichtigt.

Figur 3 zeigt eine Anordnung, bei der die in Figur 2 schematisch dargestellte Konstantstromquelle im Detail ausgeführt ist. Dabei liegt in Reihe zum Lichtsender 2a die Kollektor-Emitter-Strecke eines NPN-Transistors 4a und ein Widerstand 4b.

Ferner ist die Reihenschaltung eines Widerstands 4f, einer Diode 4e und eines PNP-Transistors 4d vorgesehen, wobei diese Reihenschaltung mit der Leitung 3b direkt und mit der Leitung 3a über einen Umschalter 4g und - je nach Stellung des Umschalters 4g - über einen Widerstand 4c oder einen Widerstand 4h verbunden ist. Der Umschalter 4g wird von einem Schwellwertschalter 4i angesteuert, der eingangsseitig an die Leitungen 3a und 3b angeschlossen ist.

Die Basis des Transistors 4a ist mit dem Verbindungspunkt des Kollektors des Transistors 4d und der Diode 4e verbunden und die Basis des Transistors 4d ist mit dem Verbindungspunkt des Lichtsenders 2a mit dem Kollektor des Transistors 4a verbunden. Damit wird der Strom durch den Transistor 4a und somit auch durch den Lichtsender 2a auf einen konstanten Wert $i_K$ geregelt, da die Spannung $U_K$ an der Leuchtdiode 2a bei konstantem Strom ebenfalls konstant bleibt.

Wenn die Spannung an den Leitungen 3a, 3b einen Wert erreicht, der den Schwellwertschalter 4i einschaltet, wird statt des Widerstandes 4c der Widerstand 4h eingeschaltet, wodurch der Betrag des Konstantstroms $i_K$ umschaltbar ist.

Figur 4 zeigt eine Anordnung, bei der zwei verschiedene Lichtsender, nämlich eine Rotlichtdiode 2e für den Meßbetrieb und eine Infrarotlichtdiode 2f für eine Referenzmessung über einen Umschalter 2d und eine Konstantstromquelle 4 wahlweise an die Verbindungsleitungen 3a und 3b anschließbar sind. Der Umschalter 2d wird von einem Zeitglied gesteuert, das aus einem an die Verbindungsleitungen 3a und 3b angeschlossenen RC-Glied mit einem Widerstand 2h und einem Kondensator 2i sowie einem an den Abgriff des RC-Gliedes angeschlossenen Schmitt-Trigger 2g besteht. Als Lichtempfänger ist zwischen den Leitungen 3a, 3b wiederum ein Fototransistor 2c mit lichtempfindlicher Kollektor-Basisdiode 2b angeschlossen.

Wenn man die Meßsonde M mit Strom oder Spannung beaufschlagt, so ist der Kondensator 2i zunächst noch ungeladen und der Schalter 2d steht in der eingezeichneten Stellung, so daß in diesem Falle die Infrarotlichtdiode 2f leuchtet. Diese Infrarotlichtdiode sendet Licht mit einer Wellenlänge aus, bei dem die Reflexion am Blut von dessen Sauerstoffsättigung unabhängig ist, jedoch von Ablagerungen auf der Meßsonde abhängt. Damit erhält man einen Referenzwert, bei dem diese Ablagerungen berücksichtigt sind und somit bei der nachfolgenden Messung zur Korrektur herangezogen werden können. Der eigentliche Meßvorgang wird ausgelöst, wenn die Spannung am Kondensator 2i den Schwellwert des Schmitt-Triggers 2g überschreitet und damit über den Umschalter 2d die Rotlichtdiode 2e eingeschaltet wird. Damit erhält man nun ein von der Blutsauerstoffsättigung abhängiges Signal.

Auch bei dieser Anordnung erweist es sich als vorteilhaft, daß dem Lichtsender, d.h. sowohl der Rotlichtdiode 2e als auch der Infrarotlichtdiode 2f eine Konstantstromquelle 4 in Serie geschaltet ist, so daß die Temperatur der Meßsonde und die Zuleitungswiderstände nicht berücksichtigt werden müssen.

Bei herkömmlichen Anordnungen liegt in Serie zum Lichtsender 2a stets ein Arbeitswiderstand, der die Stromaufteilung zwischen Lichtsender 2a und Lichtempfänger 2b steuert. Dieser Arbeitswiderstand muß verhältnismäßig groß gemacht werden, da nur dann eine ausreichende Empfindlichkeit der Meßsonde erreicht wird. Durch Einsatz einer Konstantstromquelle in Reihe zum Lichtsender 2a gemäß der Erfindung erübrigt sich dieser Arbeitswiderstand. Die Spannungsverluste an der Konstantstromquelle sind gegenüber einem entsprechenden Arbeitswiderstand deutlich verringert, was neben der Stromersparnis durch Wegfall der Referenzmessung zu einer weiteren Stromersparnis führt.

## Patentansprüche

1. Meßvorrichtung zur intrakardialen Erfassung der Blutsauerstoffsättigung zur Frequenzregelung eines Herzschrittmachers mit einer Meßsonde (M), die einen Lichtsender (2a) und einen Lichtempfänger (2b, 2c) enthält, wobei der Lichtempfänger (2b, 2c) das vom Lichtsender (2a) ausgesandte und vom Blut reflektierte Licht empfängt und wobei Lichtsender (2a) und Lichtempfänger (2b, 2c) parallel über zwei gemeinsame Leitungen (3a, 3b) an eine Auswerteschaltung (1) angeschlossen sind, dadurch gekennzeichnet, daß in Reihe zum Lichtsender (2a) eine Konstantstromquelle (4) angeordnet ist.

2. Meßvorrichtung nach Anspruch 1, dadurch gekennzeichnet, daß die Konstantstromquelle (4) auf unterschiedliche Stromwerte umschaltbar ist.

3. Meßvorrichtung nach Anspruch 1 oder 2, dadurch gekennzeichnet, daß der Lichtsender (2a) aus einer Rotlichtdiode (2e) und einer Infrarotlichtdiode (2f) besteht, wobei die Wellenlänge des von der Infrarotlichtdiode (2f) ausgestrahlten Lichts in einem Bereich liegt, bei dem dessen Reflexion am Blut von der Blut-Sauerstoffsättigung unabhängig ist und daß die Infrarotlichtdiode (2f) und die Rotlichtdiode (2e) über einen Umschalter (2d) in Reihe mit einer Konstantstromquelle dem Lichtempfänger (2m, 2k) parallel geschaltet sind.

**Claims**

1. Measuring device for the intracardial acquisition of the blood-oxygen saturation for the frequency regulation of a heart pacemaker, having a measuring probe (M) which contains a light transmitter (2a) and a light receiver (2b, 2c), the light receiver (2b, 2c) receiving the light emitted from the light transmitter (2a) and reflected from the blood, and the light transmitter (2a) and light receiver (2b, 2c) being connected in parallel by way of two common lines (3a, 3b) to an evaluation circuit (1), characterised in that a constant-current source (4) is arranged in series with the light transmitter (2a).

2. Measuring device according to claim 1, characterised in that the constant-current source (4) can be switched over to different current values.

3. Measuring device according to claim 1 or 2, characterised in that the light transmitter (2a) consists of a red light diode (2e) and an infrared light diode (2f), the wavelength of the light emitted from the infrared light diode (2f) lying in a range in which its reflection at the blood is independent of the blood-oxygen saturation, and in that the infrared light diode (2f) and the red light diode (2e) are connected by way of a change-over switch (2d) in series with a constant-current source in parallel with the light receiver (2m, 2k).

**Revendications**

1. Dispositif de mesure pour réaliser la détection intracardiaque de la saturation en oxygène du sang pour le réglage de la fréquence d'un stimulateur cardiaque, et comportant une forme de mesure (M), qui contient une source de lumière (2a), un récepteur de lumière (2b, 2c), le récepteur de lumière (2b, 2c) recevant la lumière émise par la source de lumière (2a) et réfléchie par le sang, et la source de lumière (2a) et le récepteur de lumière (2b, 2c) étant raccordés parallèlement, par l'intermédiaire de deux lignes communes (3a, 3b) à un circuit d'évaluation (1), caractérisé par le fait qu'une source de courant constant (4) est branchée en série avec l'émetteur de lumière (2a).

2. Dispositif de mesure suivant la revendication 1, caractérisé par le fait que la source de courant constant (4) peut être commutée sur différentes valeurs du courant.

3. Dispositif de mesure suivant la revendication 1 ou 2, caractérisé par le fait que la source de lumière (2a) est constituée par une diode (2e) émettant dans le rouge et d'une diode à infrarouge (2f), la longueur d'onde de la lumière émise par la diode à infrarouge (2f) étant située dans une gamme, dans laquelle sa réflexion au niveau du sang est indépendante de la saturation en oxygène du sang, et que la diode à infrarouge (2a) et la diode (2a) émettant dans le rouge sont branchées en parallèle avec le récepteur de lumière (2m, 2k), par l'intermédiaire d'un commutateur (2d) branché en série avec la source de courant constant.

FIG 1

EP 0 249 681 B1

FIG 2

FIG 3

FIG 4